# EUROPEAN PATENT SPECIFICATION

(11) **EP 3 872 477 B1**
(45) Date of publication and mention of the grant of the patent: **31.01.2024**
(21) Application number: 21159799.2
(22) Date of filing: 27.02.2021
(51) Int. Cl.: G01N 21/3577, G01N 21/3581, G01N 33/28

(54) **SYSTEM AND METHOD FOR MEASUREMENT OF FLUID FUELS**
SYSTEM UND VERFAHREN ZUR MESSUNG VON FLÜSSIGEN BRENNSTOFFEN
SYSTEME ET PROCEDE POUR LA MESURE DE CARBURANTS FLUIDES

(30) Priority: 29.02.2020 PL 43308920
(43) Date of publication of application: 01.09.2021
(73) Proprietor: Instytut Wysokich Cisnien Polskiej Akademii Nauk, 01-142 Warszawa (PL)
(72) Inventor: Knap, Wojciech, 05-807 Podkowa Lesna (PL); Skotnicki, Tomasz, 02-786 Warszawa (PL)
(74) Representative: Bury, Marek

(56) References cited:
- RAYMOND ALEXANDER W ET AL: "In situ gas sensing with a 100 GHz CMOS spectrometer", 2017 IEEE AEROSPACE CONFERENCE, IEEE, 4 March 2017 (2017-03-04), pages 1-7, XP033102941, DOI: 10.1109/AERO.2017.7943881 [retrieved on 2017-06-07]
- HONGLEI ZHAN ET AL: "The spectral analysis of fuel oils using terahertz radiation and chemometric methods", JOURNAL OF PHYSICS D: APPLIED PHYSICS, INSTITUTE OF PHYSICS PUBLISHING LTD, GB, vol. 49, no. 39, 6 September 2016 (2016-09-06), page 395101, XP020309291, ISSN: 0022-3727, DOI: 10.1088/0022-3727/49/39/395101 [retrieved on 2016-09-06]

## Description

### Field of the invention

The invention concerns a measurement system and a measurement method of fluid fuels, in particular liquid, and in particular gasoline, by means of spectrometers.

### Background of the invention

Fluid fuels, in particular gasoline are easily degenerated. In the state of the art, solutions on the base of spectrum analysis allowing for a quality assessment and a degeneration detection are known. For example, in Chinese patent application CN102323235A, a quality assessment by means of spectrum analysis by spectrums comparison is disclosed.

In the state of the art numerous sensors and measurement systems allowing for spectral analysis of composition of substances are known. In particular, sensors providing an opportunity of spectrum analysis are known - subterahertz, terahertz and photonic. Such solutions were disclosed i.e. in American patent number US8748822B1, international publications of patent applications WO2005095914A1, or WO2016050577A1. For analysis of composition and contaminations of fluids, spectrometers operating in different bands are quite commonly used. Classification to terminology is conventional, yet judging by applied technology of spectrum analysis, one can distinguish subterahertz spectrometers operating in a range of about 100 GHz to 1 THz, terahertz spectrometers operating in a range of about 1 THz to 10 THz and photonic spectrometers operating in a range of far infrared, infrared, visible radiation, or even ultraviolet. For example article "In situ gas sensing with a 100 GHz CMOS spectrometer", 2017 IEEE AEROSPACE CONFERENCE, IEEE, 4 March 2017 (2017-03-04), pages 1-7 discloses the single spectrometer with semi-confocal cavity having a movable mirror and a flat mirror. The spectrometer is enclosed in a vacuum chamber with pumps and a gas port. The pressure is monitored using a vacuum gauge. Gases are flowed continuously through the chamber, and the pressure is controllable in increments of about 1-5 mTorr using a single needle valve. In another article entitled "The spectral analysis of fuel oils using terahertz radiation and chemometric methods" JOURNAL OF PHYSICS D: APPLIED PHYSICS, INSTITUTE OF PHYSICS PUBLISHING LTD, GB, vol. 49, no. 39, 6 September 2016 (2016-09-06), page 395101, a spectral analysis of fuel oils using terahertz radiation is disclosed. A THz- time domain conventional spectroscopy system with single spectrometer is disclosed.

Drawbacks of spectrum analysis include its sensitivity to measurement conditions - in particular pressure. It is troublesome when measuring gaseous fuels, in particular under conditions of carrying out measurements in gas pipelines.

Drawbacks of spectrum analysis of liquids include impossibility of identification of components due to diffusion of characteristic spectrums for particular components.

### Summary of the invention

It is an object of the invention to provide a measurement system suitable for a quality assessment of fluid fuel and a method of carrying out a measurement of fluid fuel.

The measurement system of fluid fuels according to the invention is defined in claim 1. Thanks to such configuration, by means of the terahertz and subterahertz spectrometer, it is possible to carry out accurate measurements of spectrometry of gas collected to the measurement chamber or gas obtained from evaporation of liquid fuel, simultaneously ensuring stable measurement conditions independent from state of gas in the line and optimized for the sensitivity.

The measurement method of fluid fuels according to the invention is defined in claim 2.

The result can be compared to a reference absorption spectrum measured by other methods and a warning message can be displayed, if a discrepancy between measurement and the reference spectrum meets a predefined criterion.

Advantageously the measurement can be at carried out by opening the first valve releasing fuel vapours, then the measurements are carried out with the spectrometers and a result is recorded, whereupon the second valve is opened and by means of the pump, fuel vapours are pumped out from the chamber of the sensor unit.

Alternatively, by measuring pressure continuously by means of a manometer, an opening of the first valve and operation of the pump and the second valve can be regulated in order to steadily achieve vacuum state with dynamic flow in the measuring chamber of the sensor unit. Dynamic flow is constituted by fuel vapours, which change from liquid to gaseous state upon exposure of small amount of fluid transferring via the valve in the chamber of the sensor unit to the state close to vacuum.

Advantageously, two spectrometers are used, by means of which the measurement is carried out sequentially.

Advantageously, at least one spectrometer constitutes the semiconductor subterahertz spectrometer, with an operating band falling within the range of 100 GHz to 1000 GHz and the measurement by means of this spectrometer is carried out as the last.

### Description of drawings

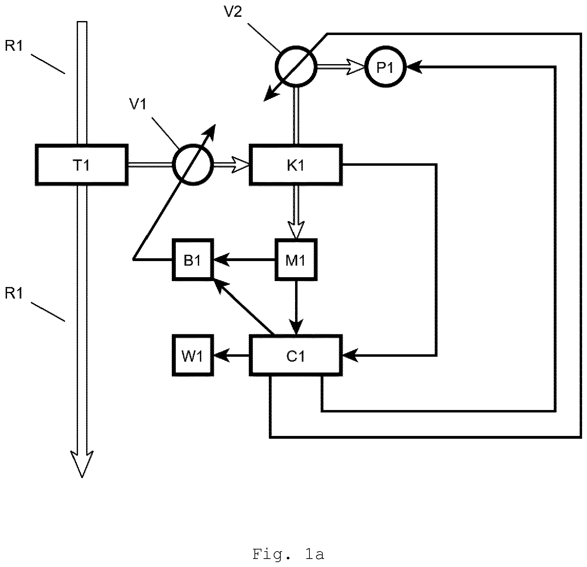

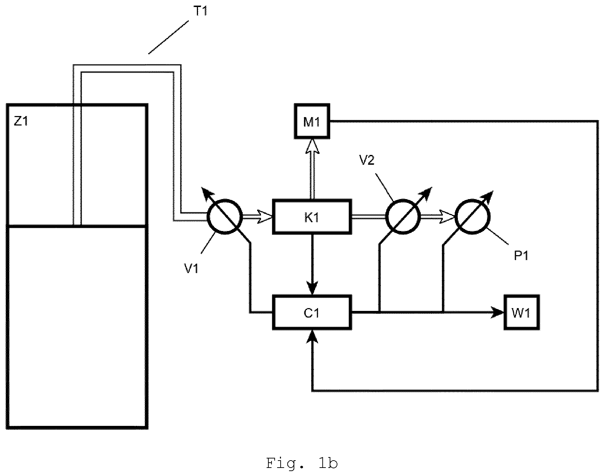

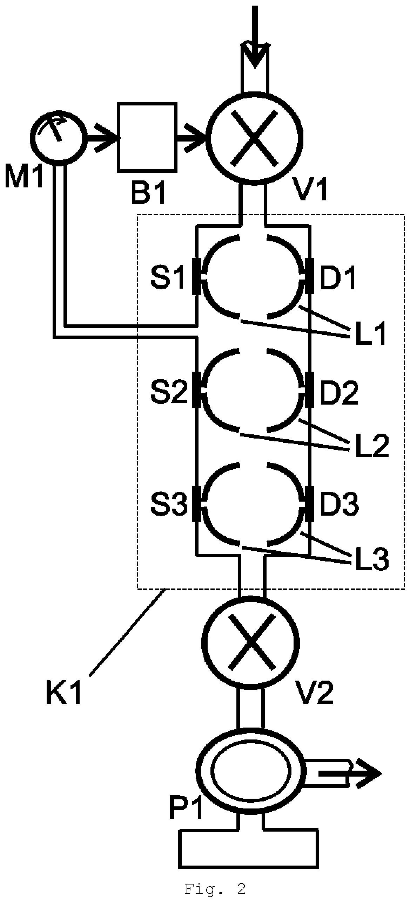

The invention will be described with reference to the following figures wherein Fig. 1a shows a block diagram of a measurement system of fluid fuels according to an embodiment of the invention, Fig. 1b shows a block diagram of a measurement system of fluid fuels according to an alternative embodiment the invention, whereas Fig. 2 schematically shows a measuring chamber with spectrometers in a sensor unit according to an embodiment of the invention (a control unit has not been shown in Fig. 2).

### Detailed description of embodiments of the invention

A block diagram of the measurement system of fluid fuels in the embodiment of the invention was shown in Fig. 1a. The system is suitable to be mounted on a line **R1** carrying fluid fuel, in particular gasoline. On the line **R1,** a branching unit **T1** is provided, discharging a part of fluid fuel and secured with the first valve **V1** constituting a needle valve. Partial opening of the first needle valve **V1** allows for introducing into the sensor unit **K1** comprising a measurement chamber with a spectrometer, gaseous fuel or liquid fuel vapours, which are subject to a spectroscopic analysis. Good effects were achieved by using a semiconductor subterahertz spectrometer with an operating band falling within the range of 100 GHz to 1000 GHz.

It was possible thanks to precision of obtaining low pressure in the measuring chamber by means of the pump **P1** placed downstream the second valve **V2.** As a result, the sensor unit **K1** is placed between two valves, the first valve **V1** and the second valve **V2,** thanks to which the measurement can be carried out under conditions set at the particular moment. For measuring pressure in the measuring chamber a manometer **M1** is used. As a first valve **V1,** the needle valve with a control unit of the valve **B1** connected to the manometer **M1** was used. Thanks to this it is possible to measure out the proper pressure from the line **R1,** specific for the measurement, regardless prevailing conditions in the line **R1:** pressure and flow rate. Thanks to this, by measuring out gas by the first valve **V1,** a result of the measurement is independent from pressure changes in the line **R1.** Using the additional pump **P1** allows for precise pressure regulation in the chamber of the sensor unit **K1.**

Pressures in the range of 5 to 20 mbar performed well. Additionally, a unit maintaining a constant temperature in the chamber of the sensor unit **K1** can be used, operating under the control of the control unit **C1.**

Good effects were obtained also with the terahertz spectrometer, with the band falling in the range of 1THz to 3THz. Terahertz spectrometers showed higher sensitivity and the ability to distinguish wider spectrum of contaminant substances, although the measurement by means of them lasts longer.

The sensor unit **K1** is connected to the control unit **C1** generating a control signal for the sensor unit **K1** and receiving the measurement result. A knowledge of a reference spectrum and an adoption of a discrepancy criterion in the way known in the state-of-the-art allows for generating a message generation by a display **W1** connected to the control unit **C1.** The control unit **C1** can be also used to control the pump **P1** and the first valve **V1** and the second valve **V2.** A coupling of the first valve with the manometer **M1** - via the control unit of the valve **B1** and/or via the control unit **C1** allows for dosing fuel subjected to the measurement by means of using the pump **P1,** what allows for repeating the measurement in pressure conditions significantly below atmospheric pressure. As an example not forming part of the present invention, it can be provided a unit cutting off fuel supply in the case of detection of a contaminant dangerous for a receiver. For this, it is sufficient to provide a controllable valve connected to the control unit **C1.**

Feeding the signal from the manometer **M1** to the control unit **C1** allows for inclusion of pressure in determining the measurement result as well as carrying out the measurement for different pressure values. The measurement system according to the invention can also be used for measuring fluid fuel in a container. Such configuration is shown on Fig. 1b. Fuel from a container **Z1** is collected by means of the branching unit **T1.** A coupling of the first valve with the manometer **M1** via a control unit **C1** allows for measuring fuel subjected to the measurement by means of the pump **P1,** what allows for repeating the measurement in conditions significantly below atmospheric pressure - in dynamic pressure conditions. In the case of fluid fuels it is so advantageous, that liquid fuel exposed to vacuum evaporates forming dynamic flow allowing for accurate and continuous measurement without broadening of spectral lines characteristic for liquids.

Described herein are the modes of operation of the system: it can operate in a continuous mode or in a sequential mode.

In the sequential mode the system operates in a cycle, in which in the chamber of the sensor unit **K1** secured from the both sides by the first valve **V1** and second valve **V2,** initially there is a vacuum, then the unit is filled with fluid measured by means of the first valve **V1** opened for the short time - if necessary, several times - up to achieving proper measuring pressure. Then, the measurement is carried out and a result is recorded, after which the second valve **V2** is opened and by means of the pump **P1** fluid is pumped out, achieving vacuum in the measuring chamber again. The measurement can be repeated, achieving a sampling effect of measurement in real time.

However, in the continuous mode, the first needle valve **V1** is opened partially, simultaneously enforcing the operation of the pump **P1.** The range of the opening of the first needle valve **V1** and pump thrust is regulated so that in the chamber of the sensor unit **K1** arises so called constant vacuum with dynamic flow. Good and stabilized value of dynamic vacuum can be obtained via electronic control of the first needle valve **V1** or both valves - the first valve **V1** and the second valve **V2.** Such configuration proved to be particularly advantageous. It can be maintained even via a simple coupling of the first valve **V1** with the manometer and simultaneously vacuum state with dynamic flow provides continuous gas exchange and stable state of favourable measurement conditions.

Reference spectrums of non-degenerated fluid fuel, in particular gasoline, can be obtained by carrying out the measurement of a calibration sample or by using measurements obtained by other methods. In a process of measurement of reference samples with different level of contamination it is easy to match permissible deviations of the spectrum from the reference.

Very good results were obtained by simultaneous using in the sensor unit two spectrometers operating in the same measuring chamber of the sensor unit **K1.** An exemplary scheme of the measuring chamber of the sensor unit **K1** with three spectrometers is shown in Fig. 2.

The measuring chamber of the sensor unit **K1** shown schematically in Fig 2 comprises a vacuum quartz tube having an internal diameter of 42 mm and a length of L = 1\m, together with quartz windows, in which sources **S1, S2, S3** and detectors **D1, D2, D3** of particular spectrometers are placed. The chambers of this type are commonly used in spectroscopy, due to low interference level, weak chemical activity and sorption activity of quartz as well as a possibility of fast and effective cleansing of the chamber during heating and vacuum pumping. In this case, some losses during transfer caused by electromagnetic wave scattering play a beneficial role, reducing an effect of interference related to multiple radiation reflections from walls and ends of the chamber.

The terahertz spectrometer can be used for example as the first spectrometer, with the source **S1** and the detector **D1,** placed next to the openings in the chamber windows of the system of the spectrometers and next to the openings in the mirrors **L1,** using signal mixing and operating in the band falling within the range of 1 THz to 2 THz. An example of this spectrometer is disclosed in the publication C. Hepp, S. Lüttjohann, A. Roggenbuck, A. Deninger, S. Nellen, T. Göbel, M. Jörger1 and R. Harig1 entitled "A cw-Terahertz Gas Analysis System with ppm Detection Limits". Such spectrometers are currently offered by the Toptica company.

As the second spectrometer, a photonic spectrometer operating in the time domain can be used, with the source **S2** and the detector **D2,** placed next to the openings in the windows of the chamber of the system of the spectrometers **K1** and next to the openings in the mirrors **L2.**

As the third spectrometer can be used for example an aforementioned semiconductor subterahertz spectrometer, with the operating band falling within the range of 100 GHz to 1000 GHz. This spectrometer allows for determining of gas concentration and fast analysis of a mixture of gases on the basis of absorption changes and radiation emission by particles included in gas composition. Radiation frequency range analyzing the gas composition is chosen so to cover characteristic absorption lines of contaminants and thus it enables identifying and differentiating those gases in contaminated hydrogen. The analysis can be carried out by using the optical chamber (optical cavity), provided in the chamber of the sensor unit **K1** located between mirrors **L3.** In the chamber controlled conditions are provided: pressure, volume, temperature suitable for absorption analysis and radiation emission. Measurement process is controlled by the control unit **C1** (not shown in Fig. 2). Absolute values of the absorption/emission coefficient are not subject to the direct measurement, but instead their changes caused by phase switching and/or radiation frequency - temporary effects. Sensitivity of spectrometers based on the temporary effects is approaching to the theoretical limits and their resolution is limited by the Doppler effect only, which is practically non-existent in the system secured with valves from the both sides - by the first valve **V1** and by the second valve **V2.** A spectrometer with a variable frequency can be used - e.g. disclosed in V. Vaks, E. Domracheva, E. Sobakinskaya, and M. Chernyaeva, "High precise terahertz spectroscopy for noninvasive medicine diagnostics," Photonics & Lasers in Medicine, vol. 3, no. 4, pp. 373-380, Sept. 2014 or a spectrometer with variable phase, e.g. disclosed in the article V. L. Vaks, E. G. Domracheva, E. A. Sobakinskaya, and M. B. Chernyaeva, "Exhaled breath analysis: physical methods, instruments, and medical diagnostics," Physics - Uspekhi, vol. 57, no. 7, pp. 684-701, July 2014.

The sources **S1, S2, S3** and the detectors **D1, D2, D3** are connected to the control unit **C1** and they are operating under its control. The connections are not shown in the figures for simplification. The control unit **C1** serves both to trigger the source operation as well as to receive and process measurement results and control operation of the valves. By using the set of the spectrometers it is important that, subterahertz spectrometer to be activated in every trial as the last because its operation activates gas and affects the operation of the remaining spectrometers.

For the spectrometer operating in sweeping frequency mode, the accuracy during determination of the instantaneous value of frequency of the radiation source shall not exceed 10⁻⁶. Such accuracy is achieved by means of the arrangement of a phased-locked loop PLL. The control signal of the frequency of the source can be placed on a dedicated reference input. In phase-locked loop of the source/generator a reference synthesizer with high frequency stability can be used. A 16-bit high speed converter can form a sweeping signal (in order to obtain a shape of control voltage close to triangular) of control frequency sweeping for signal generator submitted to duplication for achieving measuring radiation of the source S3. Module of the detector **D3** can comprise a recording camera on the basis of the waveguide detector with a Schottky diode, a low-noise preamplifier with a polarization circuit of the detector and a low-pass filter (LPF). By measuring rectified direct current (DC) of the detector (on the input of LPF), it is possible to determine intensity of spectral lines without any preliminary calibrations. The signal from the preamplifier can be sent onto the input of a very fast analog-to-digital converter, and then to a very fast digital memory, in which spectroscopic signals can be summed and averaged. Then, data can be transferred to the control unit **C1,** where averaging can be continued. Consistent signal collection allows for increasing of the signal-to-noise ratio, and therefore the sensitivity of the spectroscopic measurements.

According to one example, depending on the needs, different optical and photonic spectrometers, including infrared (near infrared and far infrared), and ultraviolet, as well as spectrometers for visible band, can be used.

The system allows for fuel collection from the container or from the line, smooth transformation to gaseous state with simultaneous convenient measurement conditions. In the case of gaseous fuels, an advantage of the system is providing convenient measurement conditions regardless
the pressure in the gas pipeline or in the container.

## Claims

1. A measurement system of fluid fuels comprising
a sensor unit **(K1)** having a tight measuring chamber with at least two sensors, a branching unit **(T1)** for supplying fluid fuel to the sensor unit **(K1),**
a pump **(P1)**, a manometer(M1) for measuring pressure in the measuring chamber,
a control unit **(C1)** for receiving at an input a signal from the sensor unit **(K1),**
wherein the sensor unit **(K1)** comprises a subterahertz semiconductor spectrometer with an operating band falling within a range of 100 GHz to 1000 GHz and a terahertz spectrometer with an operating band falling within a range of 1 THz to 3 THz,
wherein between the branching unit **(T1)** and the sensor unit **(K1)** there is a first needle valve **(V1),** coupled to the manometer (M1), whereas an outlet of the first needle valve **(V1)** is connected to an inlet of the measuring chamber of the sensor unit **(K1)** and the outlet of the measuring chamber is secured with a second controllable valve **(V2),** to which the pump **(P1)** is connected.

2. A measurement method of fluid fuels by means of at least two spectrometers, wherein the measurement of fluid fuels is carried out using the system as defined in claim 1 and the measurement is carried out by first regulating pressure conditions inside the chamber of the sensor unit **(K1)** by means of the first needle valve **(V1),** the second valve **(V2)** and the pump **(P1)**, and then
by starting the terahertz spectrometer and recording a result, and afterwards by carrying out measurement by the subterahertz sensor, with operating band falling within the range of 100 GHz to 1000 GHz as the last measurement.

3. Method according to claim 2, wherein the measurement is carried out by opening the first valve **(V1)** releasing fuel vapours, then the measurements are carried out with the spectrometers and a result is recorded, and then the second valve **(V2)** is opened and by means of the pump **(P1),** fuel vapours are pumped out from the chamber of the sensor unit **(K1)**.

4. Method according to claim 2, wherein by measuring pressure continuously by means of the manometer **(M1),** a range of opening of the first valve **(V1)** and operation of the pump **(P1)** and the second valve **(V2)** is regulated in order to achieve a state of vacuum with dynamic flow in the measuring chamber of the sensor unit.

## Patentansprüche

1. Meßsystem für flüssige Brennstoffe, umfassend
eine Sensoreinheit **(K1)** mit einer dichten Messkammer mit mindestens zwei Sensoren,
eine Abzweigungseinheit **(T1)** zum Zuführen von flüssigem Brennstoff zur Sensoreinheit **(K1),**
eine Pumpe **(P1)**
ein Manometer **(M1)** zur Messung des Drucks in der Messkammer
eine Steuereinheit **(C1),** um an einem Eingang ein Signal von der Sensoreinheit **(K1)** zu empfangen,
wobei die Sensoreinheit **(K1)** umfasst
ein Subterahertz-Halbleiterspektrometer mit einem Betriebsband, das in einen Bereich von 100 GHz bis 1000 GHz fällt, und
ein Terahertz-Spektrometer mit einem Betriebsband, das in einen Bereich von 1 THz bis 3 THz fällt,
wobei
zwischen der Verzweigungseinheit **(T1)** und der Sensoreinheit **(K1)** ein erstes Nadelventil **(V1)** vorhanden ist, das mit dem Manometer **(M1)** gekoppelt ist, wobei
ein Ausgang des ersten Nadelventils **(V1)** mit einem Eingang der Messkammer der Sensoreinheit **(K1)** verbunden ist und der Ausgang der Messkammer mit einem zweiten steuerbaren Ventil **(V2)** gesichert ist,
an das die Pumpe **(P1)** angeschlossen ist.

2. Verfahren zur Messung flüssiger Brennstoffe mittels mindestens zweier Spektrometer, wobei die Messung flüssiger Brennstoffe unter Verwendung des Systems nach Anspruch 1 durchgeführt wird und die Messung mittels dieses durchgeführt wird, indem die Bedingungen innerhalb der Kammer der Sensoreinheit **(K1)** mittels des ersten Nadelventils **(V1),** des zweiten Ventils **(V2)** und der Pumpe **(P1)** geregelt werden, und dann das Terahertz-Spektrometer gestartet und ein Ergebnis aufgezeichnet wird und danach als letztes
die Messung durch den Subterahertz-Sensor, wobei das Betriebsband in den Bereich von 100 GHz bis 1000 GHz fällt, durchgeführt wird.

3. Verfahren nach Anspruch 2, wobei die Messung durchgeführt wird, indem das erste Ventil **(V1)** geöffnet wird, das Kraftstoffdämpfe freisetzt, dann die Messungen mit den Spektrometern durchgeführt werden und ein Ergebnis aufgezeichnet wird, und dann das zweite Ventil **(V2)** geöffnet wird und mittels der Pumpe **(P1)** Kraftstoffdämpfe aus der Kammer der Sensoreinheit **(K1)** gepumpt werden.

4. Verfahren nach Anspruch 2, wobei durch kontinuierliche Druckmessung mittels des Manometers (M1) ein Öffnungsbereich des ersten Ventils **(V1)** und der Betrieb der Pumpe **(P1)** und des zweiten Ventils **(V2)** geregelt wird, um einen Unterdruckzustand mit dynamischer Strömung in der Messkammer der Sensoreinheit zu erreichen.

## Revendications

1. Système de mesure de carburants fluides comprenant
une unité de détection **(K1)** présentant une chambre de mesure étroite avec au moins deux détecteurs,
une unité de ramification **(T1)** destinée à fournir un carburant fluide à l'unité de détection **(K1),**
une pompe **(P1),**
un manomètre **(M1)** destiné à mesurer une pression dans la chambre de mesure,
une unité de commande **(C1)** destinée à recevoir au niveau d'une entrée un signal en provenance de l'unité de détection **(K1),**
dans lequel l'unité de détection **(K1)** comprend
un spectromètre à semi-conducteurs sous-térahertz avec une bande passante se trouvant dans une plage de 100 GHz à 1000 GHz et
un spectromètre térahertz avec une bande passante se trouvant dans une plage de 1 THz à 3 THz,
dans lequel
entre l'unité de ramification **(T1)** et l'unité de détection **(K1)** il y a une première soupape à pointeau **(V1),** couplée au manomètre **(M1),** tandis qu'une sortie de la première soupape à pointeau **(V1)** est raccordée à une entrée de la chambre de mesure de l'unité de détection **(K1)** et la sortie de la chambre de mesure est fixée à une seconde soupape apte à être commandée **(V2),**
à laquelle la pompe **(P1)** est raccordée.

2. Procédé de mesure de carburants fluides au moyen d'au moins deux spectromètres, dans lequel la mesure de carburants fluides est réalisée à l'aide du système tel que défini dans la revendication 1 et la mesure est réalisée par des premières conditions de pression de régulation à l'intérieur de la chambre de l'unité de détection **(K1)** au moyen de la première soupape à pointeau **(V1),** de la seconde soupape **(V2)** et de la pompe **(P1),** et ensuite en démarrant le spectromètre térahertz et en enregistrant un résultat, et par la suite en réalisant
une mesure par le détecteur sous-térahertz, avec une bande passante se trouvant dans la plage de 100 GHz à 1000 GHz en tant que dernière mesure.

3. Procédé selon la revendication 2, dans lequel la mesure est réalisée en ouvrant la première soupape **(V1)** libérant des vapeurs de carburant, ensuite les mesures sont réalisées avec les spectromètres et un résultat est enregistré, et ensuite la seconde soupape **(V2)** est ouverte et au moyen de la pompe **(P1),** des vapeurs de carburant sont évacuées de la chambre de l'unité de détection **(K1)** par pompage.

4. Procédé selon la revendication 2, dans lequel en mesurant une pression en continu au moyen du manomètre **(M1),** une plage d'ouverture de la première soupape **(V1)** et un fonctionnement de la pompe **(P1)** et de la seconde soupape **(V2)** sont régulés afin d'atteindre un état de vide avec un écoulement dynamique dans la chambre de mesure de l'unité de détection.
